# EUROPÄISCHE PATENTSCHRIFT

(11) **EP 3 561 425 B1**
(45) Veröffentlichungstag und Bekanntmachung des Hinweises auf die Patenterteilung: **20.07.2022**
(21) Anmeldenummer: 19170529.2
(22) Anmeldetag: 23.04.2019
(51) Int. Cl.: F28D 7/12, B01J 19/00, B01J 19/24

(54) **VORRICHTUNG ZUM TEMPERIEREN EINES REAKTORS**
DEVICE FOR TEMPERING A REACTOR
DISPOSITIF DE MISE EN TEMPÉRATURE D'UN RÉACTEUR

(30) Priorität: 27.04.2018 EP 18169826
(43) Veröffentlichungstag der Anmeldung: 30.10.2019
(73) Patentinhaber: Evonik Operations GmbH, 45128 Essen (DE)
(72) Erfinder: Willaert, Jan, B3920 Lommel (BE); Ludwig, Martina, 45657 Recklinghausen (DE); Geilen, Frank, 45721 Haltern am See (DE); Knossalla, Johannes, 46514 Schermbeck (DE); Quell, Thomas, 45657 Recklinghausen (DE); Franke, Robert Prof. Dr., 45772 Marl (DE)
(74) Vertreter: Evonik Patent Association

(56) Entgegenhaltungen:
- EP-A2- 0 240 340
- DE-A1- 2 157 737
- JP-A- S61 285 397
- JP-A- 2003 021 479
- US-A- 2 745 823
- US-B1- 6 838 061

## Beschreibung

Die vorliegende Erfindung betrifft eine Vorrichtung zur Temperierung eines Reaktors und ein Verfahren zur Durchführung einer chemischen Reaktion, jeweils unter Verwendung eines Wärmetauschersystems, welches ein thermisch isoliertes Innenrohr umfasst. Wärmetauschersysteme sind in der chemischen Reaktionstechnik hinlänglich bekannt und werden eingesetzt wenn Wärme aus einem Reaktor abgeführt oder in einen Reaktor eingeführt werden muss. Grundsätzlich können Wärmetauschersysteme, insbesondere bei exothermen chemischen Reaktionen eingesetzt werden, um die entstandene Reaktionswärme abzuführen und damit kühlend zu wirken. Andererseits ist es aber auch möglich, dass Wärmetauschersysteme, insbesondere bei endothermen Reaktionen dazu genutzt werden, um Wärme in den Reaktor einzuführen, damit die endotherme Reaktion durchgeführt werden kann. In diesem Fall wirken die Wärmetauschersystem heizend.

Im Stand der Technik hat es sich bewährt Wärmetauschersysteme in der Form von Rohren oder Rohrbündeln zu verwenden, wie sie beispielsweise in der EP 0 79 26 83 B1 offenbart sind. Wärmetauschersysteme umfassen üblicherweise mindestens einen Temperierfinger, der beispielsweise aus einem Doppelrohr aus einem Innen- und einem Außenrohr besteht und in den Reaktor eingelassen werden kann. Das Außenrohr des Temperierfingers steht dabei in Kontakt mit der Umgebung, insbesondere dem Reaktionsmedium. Über das Außenrohr findet daher die gewünschte Wärmeübertragung statt. Das Innenrohr dient der Zuführung des Temperiermediums, welches dann im Ringspalt zwischen äußerer Wand des Innenrohrs und innerer Wand des Außenrohrs zurückfließt. Durch das Außenrohr wird dann Wärme vom Temperiermedium auf das Reaktionsmedium oder vom Reaktionsmedium auf das Temperiermedium übertragen und so die gewünschte Wirkung, d. h. die Heiz-oder Kühlwirkung, erreicht.

Der Einsatz eines Wärmetauschersystems in einem Reaktor hat abgesehen von der eigentlichen Kühl- und/oder Heizwirkung industriell auch den Zweck das Auftreten von Temperaturunterschieden innerhalb des Reaktors so gut wie möglich zu unterbinden, damit die chemische Reaktion in jedem Teil des Reaktors ablaufen kann und sich ein möglichst hoher Stoffumsatz erzielen lässt. Ein Beispiel für einen solchen Reaktor ist im Dokument US 2 745 823 offenbart.

Ein technisches Problem bei der Verwendung der bekannten Wärmetauschersysteme besteht darin, dass der Ort der Zufuhr des Temperiermediums und der Ort mit der relativ zur Wärmeaustauschfläche größten Wärmefreisetzung oder dem größten Wärmebedarf örtlich auseinanderliegen. Das von oben in den Innenrohren der Temperierfinger zuströmende Temperiermedium wird durch das im äußeren Ringspalt entgegenströmende aufgewärmte oder abgekühlte Temperiermedium vorgewärmt oder vorgekühlt. Diese Bereiche sind nämlich durch die Wärmeleitung über das Innenrohr gekoppelt.

Bei der Verwendung von im Stand der Technik bekannten Wärmetauschersystemen kann es deshalb vorkommen, dass sich größere Temperaturunterschiede innerhalb des zu kühlenden oder zu heizenden Reaktors ausbilden, die dazu führen, dass die Ausbeute und/oder Selektivität der chemischen Reaktion sinkt. Zusätzlich besteht eine erhöhte Sensitivität gegenüber Lastwechseln. Die Aufgabe der vorliegenden Erfindung bestand darin ein neues Wärmetauschersystem bereitzustellen, bei deren Verwendung zum Temperieren einer chemischen Reaktion in einem Reaktor die vorgenannten Nachteile nicht auftreten.

Die der Erfindung zugrundeliegende Aufgabe wird durch eine Vorrichtung nach Anspruch 1 gelöst. Die Vorrichtung zum Temperieren (Abführung oder Zuführen von Wärme zum Reaktionsmedium) eines Reaktor, in dem eine exo- oder endotherme Reaktion in einem Reaktionsmedium durchgeführt werden kann, umfasst unter anderem ein Wärmetauschersystem.

Das Wärmetauschersystem der erfindungsgemäßen Vorrichtung umfasst mindestens einen Temperierfinger, welcher als Doppelrohr ausgeführt ist und aus einem thermisch isolierten Innenrohr, durch den eine Temperierflüssigkeit, in das Doppelrohr eingeleitet wird, und einem Außenrohr, welches in direktem Kontakt mit einem zu temperierenden Medium, insbesondere einem Reaktionsmedium, steht und in welchem die Temperierflüssigkeit im Ringspalt zwischen Innenrohr und Außenrohr zurückfließt, besteht. Des weiteren umfasst die erfinderische Vorrichtung eine gesteuerte Pumpe, mehrere Absperrarmaturen und Temperaturmessvorrichtungen zum Steuern der Prozesstemperatur.

Die thermische Isolation des Innenrohrs des Temperierfinger-Doppelrohrs bewirkt, dass eine Wärmeübertragung zwischen der in das Innenrohr eingeleiteten Temperierflüssigkeit und der durch den Ringspalt zurückfließenden abgekühlten oder aufgeheizten Temperierflüssigkeit minimiert bzw. vollständig unterbunden wird. Dadurch wird erreicht, dass eine Wärmeübertragung fast ausschließlich zwischen der Temperierflüssigkeit und dem Reaktionsmedium erfolgt und nicht ein Teil der Wärme zwischen einströmender und ausströmender Temperierflüssigkeit ausgetauscht wird. Da das untere Ende des Temperierfingers zumeist in dem Bereich des Reaktors angeordnet ist, wo der Großteil der chemischen Reaktion abläuft, kann durch den Einsatz des Wärmetauschersystems weiterhin erreicht werden, dass die Temperierflüssigkeit am wärmsten bzw. am kältesten Ort, d.h. dort wo der Großteil der chemischen Reaktion abläuft, die größtmögliche Wärmeaufnahmekapazität (Verwendung zum Kühlen) bzw. die größtmögliche Wärmeabgabekapazität (Verwendung zum Heizen) aufweist und nicht bereits durch eine Wärmeübertragung zwischen Innen- und Außenrohr vorgewärmt oder vorgekühlt ist.

Die Anzahl der Temperierfinger ist grundsätzlich frei wählbar und kann an den Verwendungszweck, insbesondere die zu kühlende oder zu erwärmende chemische Reaktion, und/oder an die Ausgestaltung des Einsatzortes, insbesondere das Reaktordesign, angepasst werden.

Die Ausgestaltung des Wärmetauschersystems in der erfindungsgemäßen Vorrichtung zeichnet sich insbesondere dadurch aus, dass die Wärmeleitfähigkeit des thermisch isolierten Innenrohrs des Temperierfinger-Doppelrohrs insgesamt fünffach, vorzugsweise zehnfach, kleiner ist als die Wärmeleitfähigkeit des Außenrohrs des Temperierfinger-Doppelrohrs. Der erfindungsgemäße Unterschied in den Wärmeleitfähigkeiten von Innen- und Außenrohr kann auf unterschiedlichen Wegen erzielt werden.

Eine gegenüber dem Außenrohr des Temperierfinger-Doppelrohrs bzw. gegenüber dem Material, aus dem das Außenrohr besteht, verringerte Wärmeleitfähigkeit des Innerohrs lässt sich erfindungsgemäß durch eine ein- oder beidseitige Beschichtung mit einem thermisch isolierenden Material, durch die Ausführung des Innenrohres aus einem thermisch isolierenden Vollmaterial, insbesondere einem Kunststoff, oder durch die Ausführung des Innenrohres als Doppelrohr einstellen, wobei zwischen den beiden Rohrwänden des Innendoppelrohrs ein abgedichteter Hohlraum existiert.

Das Außenrohr des Temperier-Doppelrohrs besteht vorzugsweise aus einem thermisch leitfähigen Material um eine Wärmeübertragung vom Reaktionsmedium oder auf das Reaktionsmedium zu ermöglichen. Als Materialien, aus denen das Außenrohr besteht, bieten sich insbesondere metallische Werkstoffe an, beispielsweise kohlenstoffhaltiger und/oder legierter Stahl, Nickel-Kupfer, Nickel, Nickel-Chrom-Eisen, Aluminium und Aluminiumlegierungen, Kupfer und Kupferlegierungen, Titan und Zirkon.

Die Beschichtung des Innenrohrs kann auf der inneren und/oder der äußeren Wandung des Innenrohres angebracht sein. Bei Vorliegen einer beidseitigen Beschichtung auf der inneren und der äußeren Wandung des Innenrohres des Temperierfinger-Doppelrohrs können die innere und äußere Beschichtung auch im Sinne einer Ummantelung über die Stirnfläche der Innenrohrwandung verbunden sein. Die Beschichtung besteht vorzugsweise aus einem Kunststoff und/oder einer Keramik. Der Kunststoff kann insbesondere aus der Gruppe, bestehend aus Polyurethanen, Kautschuken, Epoxidharzen, Polyamiden, Polyestern, wie Polyethylenterephthalat, Polyethern, Polyacrylaten, wie Polymethylmethacrylat, Polypyrrolen, Polyvinylestern, PTFE, PVDF, Polyolefinen, wie Polyethylen und Polypropylen, und Polyamiden, wie Nylon und PVC, ausgewählt werden. Die Keramik kann insbesondere aus der Gruppe, bestehend aus optional kristallinen Verbindungen aus metallischen oder halbmetallischen und nicht metallischen Elementen oder Mischungen daraus, beispielsweise Silikat-basierte Keramiken, wie beispielsweise Alumosilikate oder Kaoline, oxidische Keramiken, basierend auf Aluminiumoxid, Berylliumoxid, Zirconium(IV)-oxid, Titan(IV)-oxid, Aluminiumtitanat oder Bariumtitanat, oder nicht-oxidische Keramiken, basierend auf Siliziumcarbiden, Siliziumnitriden, Bornitriden, Borcarbiden, Aluminiumnitrid, Molybdändisilicid oder Wolframcarbid, ausgewählt werden. Das Beschichtungsmaterial sollte so gewählt werden, dass die Wärmeleitfähigkeit des Innenrohres insgesamt (Innenrohr und Beschichtung) kleiner ist als die Wärmeleitfähigkeit des Außenrohres. Gleichzeitig muss ein Material gewählt werden, welches die im Wärmetauschersystem vorherrschenden Temperaturen, insbesondere der Temperierflüssigkeit, aushält und welches gegenüber der Temperierflüssigkeit chemisch beständig, bestenfalls inert ist.

Weiterhin ist es möglich die Anforderungen an die Wärmeleitfähigkeit des Innenrohrs dadurch zu erfüllen, dass das Innenrohr vollständig aus einem isolierenden Vollmaterial besteht. Dazu eignen sich vorzugsweise ebenfalls Kunststoffe und/oder Keramiken. Der Kunststoff kann insbesondere aus der Gruppe, bestehen aus Polyurethanen, Kautschuken, Epoxidharzen, Polyamiden, Polyestern, wie Polyethylenterephthalat, Polyethern, Polyacrylaten, wie Polymethylmethacrylat, Polypyrrolen, Polyvinylestern, PTFE, PVDF, Polyolefinen, wie Polyethylen und Polypropylen, und Polyamiden, wie Nylon und PVC, ausgewählt werden. Die Keramik kann insbesondere aus der Gruppe, bestehend aus optional kristallinen Verbindungen aus metallischen oder halbmetallischen und nicht metallischen Elementen oder Mischungen daraus, beispielsweise Silikat-basierte Keramiken, wie beispielsweise Alumosilikate oder Kaoline, oxidische Keramiken, basierend auf Aluminiumoxid, Berylliumoxid, Zirconium(IV)-oxid, Titan(IV)-oxid, Aluminiumtitanat oder Bariumtitanat, oder nicht-oxidische Keramiken, basierend auf Siliziumcarbiden, Siliziumnitriden, Bornitriden, Borcarbiden, Aluminiumnitrid, Molybdändisilicid oder Wolframcarbid, ausgewählt werden. Das Vollmaterial kann auch ein Mischsystem Keramik/Kunststoff sein oder durch Zugabe von speziell nicht-wärmeleitenden Zusätzen zu einem metallischen Material, in ausreichendem Anteil, beispielsweise Fasermaterialien, wie Glaswolle, oder Füllstoffe, wie Glasperlen, hergestellt werden. Als thermisch isolierendes Vollmaterial, aus dem das Innenrohr des Temperierfinger-Doppelrohrs besteht, sollte ein Material gewählt werden, welches die im Wärmetauschersystem vorherrschenden Temperaturen, insbesondere der Temperierflüssigkeit, aushält und welches gegenüber der Temperierflüssigkeit chemisch beständig, bestenfalls inert ist.

Eine weitere Möglichkeit stellt die Ausgestaltung des Innenrohres als Doppelrohr dar, wobei der Hohlraum gegenüber der Umgebung abgedichtet ist. Die Herstellung des als Doppelrohr ausgeführten Innenrohres kann beispielsweise so erfolgen, dass ein Rohr mit einem größeren oder kleineren Durchmesser über das Innenrohr gestülpte oder in das Innenrohr gesteckt wird und das gestülpte oder eingefügte zweite Rohr mit dem Innenrohr an der oberen und unteren Seite verbunden wird, beispielsweise durch Verkleben, Verpressen, Verschrauben oder Verschweißen. Alternativ könnte das Innenrohr in Ausführung als Doppelrohr auch über Gussverfahren bzw. Spitzgussverfahren mit entsprechender Templatierung hergestellt werden. Dadurch entsteht zwischen Innenrohr und zweitem Rohr vorzugsweise ein gleichbleibender Ringspalt, der optional mit zusätzlichen Abstandshaltern sichergestellt wird. Der Ringspalt entspricht dann dem vorgenannten Hohlraum. Dieser Hohlraum kann in einer bevorzugten Ausführungsform mit einem thermisch isolierenden Gas und/oder einem thermisch isolierenden Material gefüllt sein. Andererseits kann in dem Hohlraum auch ein Vakuum vorliegen.

Als thermisch isolierendes Gas, mit dem der Hohlraum des Innenrohr des Doppelrohrs gefüllt sein kann, kann Luft, ein Inertgas, beispielsweise Stickstoff oder ein Edelgas wie Argon, Krypton oder Xenon, Butan, Trichlormethan, 1,1,2-Trichlor-1,1,2-trifluorethan, 1,2-Dichlor-1,1,2,2-tetrafluorethan, Tetrafluorethan, Kohlendioxid, Diethylether, Isobutan, Pentan, Peroctafluorcyclobutan, Propan, Tetrafluormethan, CFC-11 oder HCFC-141b eingesetzt werden. Als thermisch isolierendes Material, welches in den Hohlraum eingefüllt werden kann, eignen sich vorzugsweise Kunststoffe, wie vorstehend definiert oder beispielsweise auch Kunststoffschäume, wie PU-Schäume oder Styropor^{®}, oder Kunststoffgranulate, und/oder Keramiken, wie vorstehend definiert, und/oder Flüssigkeiten und/oder organische Naturstoffe, beispielsweise Baumwolle und Kork.

Das thermisch isolierte Innenrohr des erfindungsgemäßen Wärmetauschers weist vorzugsweise eine Wärmeleitfähigkeit von ≤ 0,5 W/mK, vorzugsweise ≤ 0,25 W/mK, besonders bevorzugt ≤ 0,1 W/mK auf. Dies gilt unabhängig davon, welche der vorgenannten Isolationsart verwendet worden ist.

Die Temperierflüssigkeit für das Wärmetauschersystem der erfindungsgemäßen Vorrichtung kann abhängig vom Einsatzzweck und der damit benötigten Wirkung gewählt werden. Als Temperierflüssigkeit kann insbesondere Wasser, eine wässrige Lösung, insbesondere eine wässrige Salzlösung, eine wasserbasierte Mischung, insbesondere mit einem oder mehreren Alkoholen, Marlotherm^{®} oder ein Thermoöl verwendet werden.

In einer besonders bevorzugten Ausführungsform ist die Vorrichtung zum Temperieren eines Reaktors eine Vorrichtung zum Kühlen eines Reaktors. Das Wärmetauschersystem der erfindungsgemäßen Vorrichtung ist dann ein Kühlsystem mit wie vorgenannt ausgestalteten Kühlfingern, bei denen das Innenrohr thermisch isoliert ist. Durch die thermische Isolation des Innenrohres wird eine Wärmeübertragung von der durch den Ringspalt zwischen Innen- und Außenrohr zurückfließenden erwärmten Kühlflüssigkeit (entspricht der Temperierflüssigkeit) auf die neu einströmende, kältere Kühlflüssigkeit in dem Innenrohr unterdrückt.

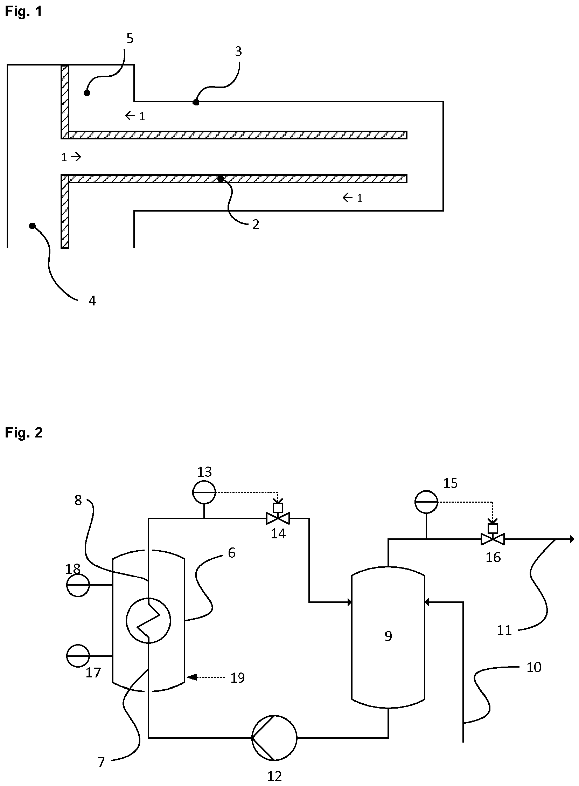

Fig. 1 zeigt ein Wärmetauschersystem der erfindungsgemäßen Vorrichtung, bei dem die Temperierflüssigkeit (1) über einen Vorlaufverteiler (4) in das Innenrohr (2) einfließt und im Ringspalt zwischen Innenrohr (2) und Außenrohr (3) wieder zum Rücklaufsammler (5) zurückfließt.

Die erfindungsgemäße Vorrichtung umfasst neben dem vorher beschriebenen Wärmetauschersystem auch einen Kreislaufbehälter, welcher die Temperierflüssigkeit, mit der das Wärmetauschersystem gespeist wird, enthält, eine erste Absperrarmatur, mit der der Temperierflüssigkeitsdruck eingestellt werden kann, eine zweite Absperrarmatur, mit der die Temperatur der Temperierflüssigkeit kontrolliert und gesteuert werden kann, und eine Pumpe, mit der die Temperierflüssigkeit im Kreislauf vom Kreislaufbehälter zum Wärmetauschersystem geführt wird und mit der die Temperierflüssigkeitsmenge im Temperierkreislauf eingestellt werden kann.

Der Reaktor in dem die exo- oder endotherme Reaktion stattfindet, d.h. in dem sich das Reaktionsmedium befindet, kann ein allgemein bekannter Reaktor sein. Der Reaktor verfügt üblicherweise über mindestens einen Einlass, über den die Reaktanden und optional ein Katalysator und/oder ein Lösemittel in den Reaktor eingebracht werden können, und mindestens einen Auslass, über den Reaktions- und Nebenprodukte aus dem Reaktor, beispielsweise zur weiteren Verarbeitung oder zu Aufarbeitung, entfernt werden können. Der Reaktor kann in einer bevorzugten Ausführungsform der vorliegenden Erfindung mindestens zwei Temperaturmessvorrichtungen umfassen, um die Reaktorinnentemperatur bzw. die Temperatur des Reaktionsmediums an verschiedenen Stellen messen zu können. Vorzugsweise ist eine der mindestens zwei Temperaturmessvorrichtungen an der Stelle des Reaktors angeordnet, wo bei einer exothermen Reaktion die meiste Reaktionswärme freigesetzt wird oder wo bei einer endothermen Reaktion die meiste (Reaktions-)Wärme benötigt wird, also die Stelle im Reaktor, wo der Großteil der chemischen Reaktion abläuft.

Fig. 2 zeigt eine beispielhafte erfindungsgemäße Vorrichtung zur Temperierung eines Reaktors (6) mit einem erfindungsgemäßen Wärmetauschersystem im Reaktor (6), der mindestens zwei Temperaturmessvorrichtungen (17, 18) umfasst. Die Temperierflüssigkeit wird aus dem Kreislaufbehälter (9) mittels einer Pumpe (12) durch eine Leitung (7) zum Wärmetauschersystem geführt und durch eine Leitung (8) zum Kreislaufbehälter (9) zurückgeführt. An der zum Kreislaufbehälter führenden Leitung (8) ist die erste Absperrarmatur (14) zur Kontrolle des Drucks angebracht. Die zweite Absperrarmatur (16) befindet sich an der Auslassleitung (11) des Kreislaufbehälters, worüber Dampf oder Flüssigkeit abgelassen werden kann. Frische Temperierflüssigkeit kann dem Kreislaufbehälter über eine Leitung (10) zugeführt werden. Die Vorrichtung kann noch weitere Messsensoren (13, 15) umfassen, über die die Absperrarmaturen gesteuert werden. Bezugszeichen (19) verdeutlicht den Zu- und/oder Ablauf der Reaktanden bzw. Produkte aus dem Reaktor (6)

Erfindungsgemäß bevorzugt ist der Reaktor ein Blasensäulenreaktor, ein Strahlenschlaufenreaktor, ein Festbettreaktor oder ein Trickle-Bed-Reaktor ist. Alle diese Reaktortypen sind hinreichend im Stand der Technik beschrieben und dem Fachmann bekannt. Ein bevorzugter Reaktortyp ist ein Blasensäulenreaktor, besonders bevorzugt ein kaskadierter Blasensäulenreaktor, der mindestens einen Einbau, insbesondere Lochplatten, aufweist. Ein solcher kaskadierte Blasensäulenreaktor wird beispielsweise in der DE 21 57 737 A offenbart. Der kaskadierte Blasensäulenreaktor weist vorzugsweise weniger als 50, besonders bevorzugt weniger als 20 Einbauten, insbesondere Lochplatten, auf.

Die Temperierflüssigkeit für die erfindungsgemäße Vorrichtung zum Temperieren eines Reaktors kann abhängig vom Einsatzzweck und der damit benötigten Wirkung gewählt werden. Als Temperierflüssigkeit kann insbesondere Wasser, eine wässrige Lösung, insbesondere eine wässrige Salzlösung, eine wasserbasierte Mischung, insbesondere mit einem oder mehreren Alkoholen, Marlotherm^{®} oder ein Thermoöl verwendet werden.

Das erfindungsgemäße Wärmetauschersystem bzw. die Temperierfinger des Wärmetauschersystems ist bzw. sind so in den Reaktor eingelassen, dass das oder die Außenrohr(e) des Wärmetauschersystems Kontakt zum Reaktionsmedium hat/haben und seine/ihre Kühl- oder Heizwirkung entfalten kann/können. Das Wärmetauschersystem mit seinem mindestens einen Temperierfinger kann von oben, von unten oder seitlich in den Reaktor eingelassen sein. Das Wärmetauschersystem ist dementsprechend mit einem Boden-, einem Deckel- oder einem seitlichen Flansch des Reaktors verbunden. Bevorzugt werden Konstruktionen verwendet, bei denen das Wärmetauschersystem mit dem Deckelflasch verbunden sind und die Temperierfinger hängend von oben in den Reaktor eingeführt werden

Das Reaktionsmedium kann prinzipiell ein- oder mehrphasig vorliegen. Das Vorhandensein eines ein- oder mehrphasigen Reaktionsmediums hängt von den Umständen der entsprechenden exo- oder endothermen chemischen Reaktion und den entsprechenden Reaktionsparametern ab. Eine bevorzugte exo- oder endotherme Reaktion, die in dem Reaktor durchgeführt werden kann, ist eine Hydroformylierung, eine Lösungspolymerisation gasförmiger Monomere, eine Selektivoxidation organischer Verbindungen, beispielsweise zur Erzeugung von Carbonsäuren, eine Hydrierung von C-C- oder C-X-Mehrfachbindungen, wobei X = O oder N ist, eine Fischer-Tropsch-Synthese, sofern in der Slurry-Phase, eine Carbonylierungsreaktion, eine Hydroaminomethylierungsreaktion, eine Hydrocyanierung, eine Hydrierung, eine Hydrosilylierung, eine Oxidation, eine Pyrolyse, eine Dampfreformierung, eine Dehydrierung, eine Dehydratisierung oder eine Oligomerisierung. Denkbar sind auch Alkoxy- oder Hydroxycarbonylierungen.

Als Kreislaufbehälter können bekannte Kreislaufbehälter verwendet werden. Die Voraussetzung ist, dass der Kreislaufbehälter gegenüber der Temperierflüssigkeit und den vorherrschenden Temperaturen (chemisch) beständig, bestenfalls inert ist. Die Größe des Kreislaufbehälters sollte an die benötigte Kühl- oder Heizwirkung der Temperierflüssigkeit im Hinblick auf die chemische Reaktion angepasst werden. Der Kreislaufbehälter umfasst mindestens einen Auslass, über den die Temperierflüssigkeit zum Wärmetauschersystem geführt wird und mindestens einen Einlass, durch den die durch den Wärmetauschersystem geführte Temperierflüssigkeit zum Kreislaufbehälter zurückgelangt. Weiterhin kann der Kreislaufbehälter noch einen weiteren Einlass, über den frische Temperierflüssigkeit hinzugefügt wird, und/oder einen weiteren Auslass aufweisen, über den beispielsweise Dampf aus dem Kreislaufbehälter abgelassen werden kann.

Die erste Absperrarmatur, mit der der Temperierflüssigkeitsdruck kontrolliert und gesteuert werden kann, dient insbesondere der Verhinderung des Verdampfens der Temperierflüssigkeit in den Temperierfingern oder den Leitungen vom und zum Wärmetauschersystem. Eine erfindungsgemäße erste Absperrarmatur kann ein Ventil, ein Schieber, ein Hahn oder eine Klappe sein, bevorzugt ist die erste Absperrarmatur ein Ventil oder ein Schieber, besonders bevorzugt ein Ventil. In einer weiterhin bevorzugten Ausführungsform wird der Druck der Temperierflüssigkeit in Abhängigkeit von einer Temperaturdifferenz (ΔT) zwischen den Reaktorinnentemperaturen an den mindestens zwei Temperaturmessvorrichtungen im Reaktor gesteuert. Dazu kann die erste Absperrarmatur in den Rohrleitungen hinter dem (stromabwärts vom) Wärmetauschersystem angeordnet sein. Ist im Hinblick auf die Temperaturdifferenz zwischen den Temperaturen an den mindestens zwei Temperaturmessvorrichtungen an den genannten Stellen im Reaktor eine Anpassung des Temperierflüssigkeitsdrucks notwendig (beispielsweise bei Über- oder Unterschreiten eines voreingestellten Grenzwertes von ΔT), kann die erste Absperrarmatur weiter geöffnet werden, um den Druck zu senken, oder weiter geschlossen werden, um den Druck zu erhöhen. Das Öffnen und Schließen kann manuell oder automatisch erfolgen, insbesondere Computer-unterstützt.

Die zweite Absperrarmatur, mit der die Temperatur (Eingangstemperatur am Wärmetauschersystem) der Temperierflüssigkeit kontrolliert und gesteuert werden kann, dient der Anpassung der Temperatur, worüber sich die Fähigkeit zur Wärmeaufnahme durch die Temperierflüssigkeit einstellen lässt. Eine erfindungsgemäße zweite Absperrarmatur kann ein Ventil, ein Schieber, ein Hahn oder eine Klappe sein, bevorzugt ist die zweite Absperrarmatur ein Ventil oder ein Schieber, besonders bevorzugt ein Ventil. In einer weiterhin bevorzugten Ausführungsform wird die Temperatur der Temperierflüssigkeit in Abhängigkeit einer Reaktorinnentemperatur, vorzugsweise von der Reaktorinnentemperatur an der Stelle im Reaktor, wo der Großteil der chemischen Reaktion abläuft, gesteuert. Dazu kann die zweite Absperrarmatur am weiteren Auslass bzw. der weiteren Auslassleitung des Kreislaufbehälters, über den beispielsweise Dampf aus dem Kreislaufbehälter abgelassen werden kann, angeordnet sein. Ist im Hinblick auf die Temperaturmessung an der Temperaturmessvorrichtung an der genannten Stelle eine Anpassung der Eingangstemperatur der Temperierflüssigkeit notwendig, kann die zweite Absperrarmatur geöffnet werden, um beispielsweise Dampf oder erhitzte Temperierflüssigkeit aus dem Kreislaufbehälter abzulassen, oder geschlossen werden um den abzulassende Menge an Dampf oder erhitzter Temperierflüssigkeit zu begrenzen oder vollständig zu unterbinden. Das Öffnen und Schließen kann manuell oder automatisch erfolgen, insbesondere Computer-unterstützt. Abgelassener Dampf oder erhitze Temperierflüssigkeit kann dann durch Hinzufügen von frischer Temperierflüssigkeit ersetzt werden.

Die Temperaturmessvorrichtungen in Figur 2 wurden willkürlich gewählt und ihre Platzierungen sind nicht einschränkend zu verstehen. Es können auch Temperaturmessvorrichtungen an anderen Stellen im Reaktor verwendet werden, sofern diese einer besseren Reaktionsführung dienlich sind.

Die Pumpe, der die Temperierflüssigkeit im Kreislauf vom Kreislaufbehälter zum Wärmetauschersystem geführt wird und mit der die Temperierflüssigkeitsmenge im Temperierkreislauf eingestellt werden kann, sorgt dafür dass die Menge der Temperierflüssigkeit entsprechend des Bedarfs angepasst werden kann. Die Pumpe in der erfindungsgemäßen Vorrichtung zum Temperieren eines Reaktors kann eine dem Fachmann bekannte Pumpe sein, die in der Lage ist, die Temperierflüssigkeit in ausreichender Menge, im entsprechendem Temperaturfenster und ausreichendem Druck durch die Leitungen zu pumpen. Die Pumpe sollte gegenüber der Temperierflüssigkeit chemisch beständig, bestenfalls inert sein.

In einer besonders bevorzugten Ausführungsform kann die erfindungsgemäße Vorrichtung zum Temperierung für die Kühlung (d.h. zur Abführung von Reaktionswärme) eines Reaktors, in dem eine exotherme Reaktion in einem Reaktionsmedium durchgeführt werden kann oder durchgeführt wird, verwendet werden. Die Vorrichtung kann einen Reaktor, das vorgenannt erwähnte Wärmetauschersystem mit einem oder mehreren Kühlfinger(n) und einer entsprechenden Kühlflüssigkeit, einen Kreislaufbehälter, welcher die Kühlflüssigkeit enthält, mit der das Wärmetauschersystem gespeist wird, eine erste Absperrarmatur, mit der der Kühlflüssigkeitsdruck eingestellt werden kann, eine zweite Absperrarmatur, mit der die Temperatur der Kühlflüssigkeit kontrolliert und gesteuert werden kann, und eine Pumpe, mit der die Kühlflüssigkeit im Kreislauf vom Kreislaufbehälter zum Wärmetauschersystem geführt wird und mit der die Kühlflüssigkeitsmenge im Kühlkreislauf eingestellt werden umfassen.

Der Reaktor ist vorzugsweise ein Blasensäulenreaktor, ein Strahlenschlaufenreaktor, ein Festbettreaktor oder ein Trickle-Bed-Reaktor ist. Ein bevorzugter Reaktortyp ist ein Blasensäulenreaktor, besonders bevorzugt ein kaskadierter Blasensäulenreaktor, der mindestens einen Einbau, insbesondere Lochplatten, aufweist. Der kaskadierte Blasensäulenreaktor weist vorzugsweise weniger als 50, besonders bevorzugt weniger als 20 Einbauten, insbesondere Lochplatten, auf.

Weiterhin bevorzugt wird als Kühlflüssigkeit eine wasserbasierte Kühlflüssigkeit, insbesondere Wasser verwendet. Erfindungsgemäß bevorzugt liegt eine Druckwasserkühlung und keine Siedewasserkühlung vor.

Die Siedekühlung ist ein gängiges Konzept für die Kühlung exothermer Reaktionen. Dabei wird flüssiges Kühlwasser in den Wärmetauscher eingespeist. An den Wandungen, die mit dem Reaktionsmedium in Kontakt stehen, wird dabei das Wasser zunächst erwärmt, wobei die aufgenommene Wärmemenge von der Temperaturdifferenz und Wärmekapazität des Kühlwassers abhängt. In der Regel wird das Wasser für die Siedekühlung mit einer Temperatur knapp unter der Siedetemperatur eingebracht, weshalb es bereits nach Aufnahme einer geringen Wärmemenge zur Verdampfung des Wassers kommt. Die mögliche Wärmeaufnahme ist dabei jedoch durch die Verdampfungsenthalpie des Wassers bei den vorherrschenden Bedingungen (Temperatur, Druck) begrenzt. Die Siedetemperatur des Wassers kann beispielsweise über den Druck im Kreislaufbehälter eingestellt.

Im dem Teil des Reaktors, wo aufgrund der höchsten Konzentration an Reaktanden/Edukten ein Großteil der Reaktion abläuft, wird auch der Großteil der Reaktionswärme freigesetzt, die abgeführt werden muss. In diesem Bereich befindet sich üblicherweise das untere Ende des Temperier- bzw. Kühlfingers. Dieser Bereich unterliegt weiterhin einer besonderen Vakanz bei der Temperatursteuerung des Gesamtreaktors, da die bei manchen chemischen Reaktionen eingesetzten Katalysatoren (insbesondere bei homogen katalysierten Reaktionen wie der Hydroformylierung) bei zu hohen Temperaturen einer thermischen Zersetzung unterliegen können. Die maximale Temperatur für diese unteren Zonen wird folglich durch die Zersetzungstemperatur des Katalysators begrenzt. In der Regel wird die Reaktortemperatur möglichst nah an die für die Katalysatorstabilität maximal zulässige Temperatur herangefahren, da hohe Temperaturen sowohl die Reaktionsgeschwindigkeit, als auch die Produktqualität begünstigen.

Diese Katalysatorstabilitäts-gekoppelte Temperatur-Limitierung, die abzuführende Wärmemenge und die energetisch gekoppelte Mediumszu- und -abfuhr im Wärmetauscher (für die Kühlung) führen nun dazu, dass die Temperatur der Kühlflüssigkeit, insbesondere Wasser, über den gesamten Bereich der Kühlfläche konstant bleiben kann. In den Reaktorabschnitten mit weniger Wärmetönung kann diese Temperatur nicht ohne Einflussnahme auf den Gesamtprozess angepasst werden, d.h. die Temperatur in einem Abschnitt des Reaktors kann nicht ohne Einfluss auf den gesamten Reaktor eingestellt werden. Die entsprechend der verfügbaren Wärmeaustauschfläche und der Betriebsparameter eingestellte Vorlauftemperatur des Kühlwassers führt dazu, dass die Temperatur der Kühlflüssigkeit auf der gesamten Länge des Wärmetauschers vorherrschend ist. In Teilen des Reaktors, in welchem aufgrund der durch vorherigen Umsatz bereits verringerten Eduktkonzentration (und/oder der Tatsache, dass zum Teil nur noch schwer umzusetzende Eduktisomere vorhanden sind) deutlich weniger Wärme freigesetzt wird, ist nun das Verhältnis zwischen Wärmeaustauschfläche und Kühlwassertemperatur ungünstig. Das Reaktionsgemisch kann deutlich stärker auskühlen, als es für einen effizienten Reaktorbetrieb zielführend wäre. So werden in mit Siedekühlung gekühlten Reaktoren abweichend von einer isothermen Betriebsweise Temperaturunterschiede entlang des Reaktors (Zu- und Ablauf des Reaktionsmediums) von bis zu 20K oder mehr beobachtet. Die geringere Temperatur in einem Teil des Reaktors verlangsamt die ohnehin geringere Reaktionsgeschwindigkeit weiter und hat obendrein einen negativen Einfluss auf die Produktqualität.

Die Druckwasserkühlung, bei der man das Sieden der Flüssigkeit durch einen ausreichend hohen Überdruck unterdrückt, benötig grundsätzlich entweder eine höhere Temperaturdifferenz zwischen Reaktionsmedium und Kühlwasser oder eine sehr viel höhere Kühlwassermenge, die durch den Wärmeaustauscher geleitet werden muss. Der Grund dafür ist der Unterschied zwischen der Verdampfungsenthalpie von Wasser und der spezifischen Wärmekapazität von Wasser bei einer gegebenen Temperatur, weswegen in technischen Anlagen üblicherweise eine Siedekühlung bevorzugt wird. Überraschenderweise hat sich gezeigt, dass eine Umstellung von einer Siedekühlung zu einer Druckwasserkühlung einen zusätzlichen positiven Effekt auf die Umsatzleistung von chemischen Reaktionen, insbesondere Hydroformylierungsreaktionen, haben kann.

Die Verwendung einer Druckwasserkühlung in Verbindung mit einem thermisch isolierten Wärmetauscher, der wie vorgenannt ausgestaltet sein kann, ermöglicht eine angenähert isotherme Betriebsweise über den gesamten Reaktor. Unter angenähert isotherm soll verstanden werden, dass die Temperaturdifferenz zwischen wärmster und kältester Stelle im Reaktor höchstens halb so groß wie mit Siedekühlung und insbesondere kleiner als 13 K, vorzugsweise kleiner als 7,4 K, besonders bevorzugt kleiner als 5 K ist.

Die vorliegende Erfindung betrifft weiterhin ein Verfahren zur Durchführung exothermer chemischer Reaktionen in einem Reaktor, wobei die Reaktionswärme durch ein innenliegendes Wärmetauschersystem, wie vorstehend definiert abgeführt wird, wobei die das Wärmetauschersystem durchströmende Kühlflüssigkeit nicht siedet und die Temperaturdifferenz zwischen kältester und wärmster Stelle im Reaktor kleiner als 13 K, vorzugsweise kleiner als 7,4 K und besonders bevorzugt kleiner als 5 K ist.

Die exotherme chemische Reaktion ist vorzugsweise eine Hydroformylierung. Bei der Hydroformylierung werden vorzugsweise Olefine mit 2 bis 20 Kohlenstoffatomen mit einer Mischung aus Wasserstoff und Kohlenmonoxid - dem sogenannten Synthesegas - zu Aldehyden mit einem Kohlenstoffatom mehr umgesetzt. Die Aldehyde können beispielsweise durch Hydrierung in Alkohole überführt werden.

Als Katalysator können in der Hydroformylierung unter anderem Metallverbindungen oder metallorganische Komplexverbindungen genutzt werden, die ein Metall als Zentralatom aufweisen, welches mit verschiedenen Liganden komplexiert sein kann. Erfindungsgemäß bevorzugt sind Hydroformylierungen mit Co-, Rh-, Ru-, Ir-, Pd- oder Fe-Katalysatoren, wobei die Rh-, Ru-, Ir-, Pd- oder Fe-Atome besonders bevorzugt Liganden aufweisen

Als Liganden können Organophosphor-Verbindungen eingesetzt werden, nicht limitierende Beispiele sind Organophosphine oder Organophosphite. Ein solches Katalysatorsystem kann in dem flüssigen Reaktionsgemisch aus Olefin und darin gelösten Synthesegas gelöst werden und ist damit vorzugsweise homogen. Die Abtrennung des Katalysators kann aus dem abgeführten Reaktionsgemisch, welches üblicher Weise die gebildeten Aldehyde, Nebenprodukte, nicht umgesetzte Edukte und den gelösten Katalysator oder Bestandteile oder Abbauprodukte desselben, also reines Metall, freie Liganden oder degeneriertes Metall und Liganden enthält, erfolgen.

Insbesondere bevorzugt sind Katalysatorsysteme, die Cobalt oder Rhodium als Zentralatom aufweisen, wobei letztere oft mit Organophosphor-Liganden wie Phosphin-, Phosphit- oder Phosphoramidit-Verbindungen komplexiert sein können.

Da insbesondere Rhodiumkatalysatoren mit Organophosphorliganden aufgrund hoher Marktpreise des Rhodiummetalles und oft aufwendiger Synthese der Liganden recht kostspielig sind, wird in der industriellen Technik ein großer Aufwand betrieben um die Aktivität der Katalysatoren möglichst lange aufrecht zu erhalten. Die katalytisch aktiven Komplexe unterliegen in der Regel sowohl während der Reaktion, als auch während der Aufarbeitung der katalysatorhaltigen Reaktionsgemische komplexen, von Druck (vor allem Kohlenmonoxidpartialdruck) und Temperatur abhängigen chemischen Gleichgewichten. Erschwerend kommt hinzu, dass die zum KatalysatorKomplex gehörigen Organophosphor-Liganden sehr empfindlich auf Zustandsänderungen reagieren und mitunter rasch degenerieren. Da die Zersetzungsprodukte der Liganden entweder die metallorganischen Komplexe nicht mehr zu stabilisieren vermögen oder aber besonders stark an die Metallzentren binden, wird das empfindliche, für die erfolgreiche katalytische Reaktion notwendige, Gleichgewicht am Katalysatorkomplex gestört. Dies führt makroskopisch zu einer Desaktivierung des Katalysators. Ein desaktivierter Katalysator ist bestenfalls nur aufwändig zu reaktivieren. Da vor allem die als besonders aktive Liganden bekannten Organophosphite bei höheren Temperaturen verstärkt Zersetzungsreaktionen unterliegen, ist eine gesteuerte Temperaturführung während der exothermen chemischen Reaktion der Hydroformylierung unerlässlich.

Zur Abführung der freiwerdende Reaktionswärme der Hydroformylierung sind die Reaktoren mit den erfindungsgemäßen Wärmetauschern, wie vorstehend definiert, oder mit der erfindungsgemäßen Vorrichtung zur Temperierung, wie vorstehend definiert, ausgestattet.

Bei der Hydroformylierung handelt es sich um eine Reaktion, bei der das Reaktionsgemisch vorzugsweise aus zumindest zwei Phasen besteht. Die Alkene können als flüssige Phase in den Reaktor eingebracht oder werden in dem oder vor dem Reaktor in einem organischen Lösungsmittel gelöst, wodurch sich eine flüssige Phase im Reaktor ergibt. Das Synthesegas wird üblicherweise gasförmig in den Reaktor eingebracht. Da das Synthesegas für die Reaktion mit den Alkenen und den ebenfalls in der flüssigen Phase gelösten Katalysatoren in Kontakt kommen muss, ist es nötig, dass die Komponenten des Synthesegases in der flüssigen Phase gelöst werden. Da der Stoffübergang von der Gas- in die Flüssigphase maßgeblich von der Größe der Phasengrenzfläche beeinflusst wird, ist man bestrebt eine möglichst hohe Dispersion des Gases in der Flüssigphase zu erzielen. Um das zu ermöglichen ist der Reaktor vorzugsweise ein Blasensäulenreaktor oder ein Strahlenschlaufenreaktor. Ein bevorzugter Reaktortyp ist ein Blasensäulenreaktor, besonders bevorzugt ein kaskadierter Blasensäulenreaktor, der mindestens einen Einbau, insbesondere Lochplatten, aufweist. Der kaskadierte Blasensäulenreaktor weist vorzugsweise weniger als 50, besonders bevorzugt weniger als 20 Einbauten, insbesondere Lochplatten, auf.

Die Hydroformylierung kann bei einem Druck von 10 bis 400 bar, vorzugsweise 15 bis 250 bar durchgeführt werden. Die Temperatur bei der Hydroformylierung kann 70 bis 250 °C, vorzugsweise 100 bis 200 °C betragen.

Erfindungsgemäß besonders bevorzugt ist eine Vorrichtung zur Hydroformylierung von Olefinen mit 2 bis 20 Kohlenstoffatomen, die einen, optional kaskadierten Blasensäulenreaktor mit innenliegendem erfindungsgemäßen Wärmetauscher mit mindestens einem Kühlfinger, der als Doppelrohr aus Innenrohr und Außenrohr ausgeführt ist, wobei das Innenrohr wie vorstehend definiert thermisch isoliert ist, für die Kühlung des Reaktors bzw. des Reaktionsmediums umfasst. Die Kühlflüssigkeit ist vorzugsweise Wasser. Darüber hinaus verfügt die erfindungsgemäße Vorrichtung zur Hydroformylierung von Olefinen über einen Kreislaufbehälter, eine Pumpe, eine erste Absperrarmatur, mit der verhindert werden kann, dass das Kühlmittel in den Kühlrohren verdampft und eine zweite Absperrarmatur, mit der Eingangstemperatur der Kühlflüssigkeit reguliert werden kann. In einer besonderen Ausführungsform wird die Kühlwassereingangstemperatur über zweite Absperrarmatur des Kreislaufbehälters in Abhängigkeit einer Reaktorinnentemperaturmessung mittels einer Temperaturmessvorrichtung gesteuert. In einer weiteren besonders bevorzugten Ausführungsform der Erfindung wird zusätzlich der Druck der Temperierflüssigkeit über die erste Absperrarmatur in Abhängigkeit von einer Temperaturdifferenz (ΔT) zwischen zwei Temperaturmessvorrichtungen im Reaktor geregelt.

## Patentansprüche

1. Vorrichtung zur Temperierung eines Reaktors, in dem eine exo- oder endotherme Reaktion in einem Reaktionsmedium durchgeführt werden kann, wobei die Vorrichtung einen Reaktor (6), ein Wärmetauschersystem, einen Kreislaufbehälter (9), welcher eine Temperierflüssigkeit enthält, mit der das Wärmetauschersystem gespeist wird, und eine Pumpe (12), mit der die Temperierflüssigkeit durch eine Leitung (7) zum Wärmetauschersystem geführt und durch eine Leitung (8) zum Kreislaufbehälter (9) zurückgeführt wird und mit der die Temperierflüssigkeitsmenge im Temperierkreislauf eingestellt wird, umfasst,
wobei das Wärmetauschersystem mindestens einen Temperierfinger umfasst, wobei der Temperierfinger ein Doppelrohr ist, bestehend aus einem Innenrohr (2), durch den eine Temperierflüssigkeit (1) in das Doppelrohr eingeleitet wird und einem Außenrohr (3), welches in direktem Kontakt mit dem Reaktionsmedium steht und in welchem die Temperierflüssigkeit (1) im Ringspalt zwischen Innenrohr (2) und Außenrohr (3) zurückfließt, wobei das Wärmetauschersystem so in den Reaktor eingelassen ist, dass das/die Außenrohr/e des Temperierfingers Kontakt zum Reaktionsmedium hat/haben,
**dadurch gekennzeichnet, dass** das Innenrohr (2) des Doppelrohrs thermisch isoliert ist,
und, dass die Vorrichtung eine erste Absperrarmatur (14), mit der der Temperierflüssigkeitsdruck eingestellt wird, eine zweite Absperrarmatur (16), mit der die Temperatur der Temperierflüssigkeit kontrolliert und gesteuert wird, und mindestens zwei Temperaturmessvorrichtungen (17, 18) zur Messung der Reaktorinnentemperatur umfasst,
wobei die erste Absperrarmatur (14) an der zum Kreislaufbehälter führenden Leitung (8) angebracht ist;
wobei die zweite Absperrarmatur (16) sich an einer Auslassleitung (11) des Kreislaufbehälters befindet, über die Dampf oder Flüssigkeit abgelassen werden kann; und
wobei die Temperatur der Temperierflüssigkeit in Abhängigkeit einer Reaktorinnentemperatur gesteuert wird

2. Vorrichtung zur Temperierung eines Reaktors nach Anspruch 1, wobei das Innenrohr (2) des Doppelrohrs des Wärmetauschers durch eine ein- oder beidseitige Beschichtung mit einem thermisch isolierenden Material, insbesondere einem Kunststoff und/oder einer Keramik, durch die Ausführung des Innenrohres aus einem thermisch isolierenden Vollmaterial, insbesondere einem Kunststoff und/oder einer Keramik, oder durch die Ausführung des Innenrohres als Doppelrohr, wobei zwischen den beiden Rohrwänden des Innendoppelrohrs ein abgedichteter Hohlraum existiert, ausgebildet wird.

3. Vorrichtung zur Temperierung eines Reaktors nach Anspruch 2, wobei der Hohlraum zwischen den beiden Rohrwänden des Innendoppelrohrs des Wärmetauschers mit einem thermisch isolierenden Gas und/oder einem thermisch isolierenden Material gefüllt ist oder in dem Hohlraum ein Vakuum vorliegt.

4. Vorrichtung zur Temperierung eines Reaktors nach einem der Ansprüche 1 bis 3, wobei die Temperatur der Temperierflüssigkeit in Abhängigkeit von der Reaktorinnentemperatur an der Stelle im Reaktor, wo der Großteil der chemischen Reaktion abläuft, gesteuert wird.

5. Vorrichtung zur Temperierung eines Reaktors nach einem der Ansprüche 1 bis 4, wobei der Druck der Temperierflüssigkeit in Abhängigkeit von einer Temperaturdifferenz (ΔT) zwischen den Reaktorinnentemperaturen an den mindestens zwei Temperaturmessvorrichtungen (17, 18) im Reaktor gesteuert.

6. Vorrichtung zur Temperierung eines Reaktors nach einem der Ansprüche 1 bis 5, wobei als Kühlflüssigkeit Wasser, eine wässrige Lösung, insbesondere eine wässrige Salzlösung, eine wasserbasierte Mischung, insbesondere mit einem oder mehreren Alkoholen, Marlotherm^{®} oder ein Thermoöl verwendet wird.

7. Vorrichtung zur Temperierung eines Reaktors nach einem der Ansprüche 1 bis 6, wobei der Reaktor ein Blasensäulenreaktor, ein Strahlenschlaufenreaktor, ein Festbettreaktor oder ein Trickle-Bed-Reaktor ist.

8. Vorrichtung zur Temperierung eines Reaktors nach einem der Ansprüche 1 bis 7, wobei die exotherme Reaktion eine Hydroformylierung, eine Lösungspolymerisation gasförmiger Monomere, eine Selektivoxidation organischer Verbindungen, beispielsweise zur Erzeugung von Carbonsäuren, eine Hydrierung von C-C- oder C-X-Mehrfachbindungen, wobei X = O oder N ist, eine Fischer-Tropsch-Synthese, sofern in der Slurry-Phase, eine Carbonylierungsreaktion, eine Hydroaminomethylierungsreaktion, eine Hydrocyanierung, eine Hydrierung, eine Hydrosilylierung, eine Oxidation, eine Pyrolyse, eine Dampfreformierung, eine Dehydrierung, eine Dehydratisierung oder eine Oligomerisierung ist.

9. Vorrichtung zur Temperierung eines Reaktors nach einem der Ansprüche 1 bis 8, wobei das Wärmetauschersystem von unten, von oben oder seitlich in den Reaktor geführt wird und entsprechend mit dem Boden-, Deckel- oder seitlichen Flansch des Reaktors verbunden ist, vorzugsweise von oben in den Reaktor geführt wird und mit dem Deckelflansch verbunden ist.

10. Verwendung der Vorrichtung zur Temperierung eines Reaktors nach einem der Ansprüche 1 bis 9 für die Kühlung eines Reaktors, in dem eine exotherme Reaktion durchgeführt werden kann.

11. Verfahren zur Durchführung exothermer chemischer Reaktionen in einem Reaktor, wobei die Reaktionswärme durch die Vorrichtung zur Temperierung eines Reaktors nach einem der Ansprüche 1 bis 9 abgeführt wird, wobei die das Wärmetauschersystem durchströmende Kühlflüssigkeit nicht siedet und die Temperaturdifferenz zwischen kältester und wärmster Stelle im Reaktor kleiner als 13 K ist.

12. Verfahren nach Anspruch 11, wobei die exotherme chemische Reaktion eine Hydroformylierung mit Co-, Rh-, Ru-, Ir-, Pd- oder Fe-Katalysatoren ist.

13. Verfahren nach Anspruch 12, wobei die Temperaturdifferenz zwischen kältester und wärmster Stelle im Reaktor (bei Durchführung der Reaktion) kleiner als 13 K, vorzugsweise kleiner als 7,4 K und besonders bevorzugt kleiner als 5 K ist.

## Claims

1. Apparatus for controlling the temperature of a reactor in which an exo- or endothermic reaction can be conducted in a reaction medium, wherein the apparatus comprises a reactor (6), a heat exchanger system, a circulation vessel (9) containing a temperature control fluid with which the heat exchanger system is fed, and a pump (12) with which the temperature control fluid is returned via a conduit (7) to the heat exchanger system and via a conduit (8) to the circulation vessel (9), and with which the volume of temperature control fluid in the temperature control circuit is adjusted,
wherein the heat exchanger system comprises at least one temperature control finger, wherein the temperature control finger is a double tube consisting of an inner tube (2) through which a temperature control fluid (1) is introduced into the double tube and an outer tube (3) which is in direct contact with the reaction medium and in which the return flow of the temperature control fluid (1) is in the annular gap between inner tube (2) and outer tube (3), wherein the heat exchanger system is recessed into the reactor such that the outer tube(s) of the temperature control finger are in contact with the reaction medium,
**characterized in that** the inner tube (2) of the double tube is thermally insulated
and **in that** the apparatus has a first shut-off fitting (14) with which the temperature control fluid pressure can be adjusted, a second shut-off fitting (16) with which the temperature of the temperature control fluid can be monitored and controlled, and at least two temperature measurement devices (17, 18) for measuring the internal reactor temperature, wherein the first shut-off fitting (14) is mounted on the conduit (8) that leads to the circulation vessel;
wherein the second shut-off fitting (16) is present in an outlet conduit (11) of the circulation vessel, by means of which steam or liquid can be discharged; and
wherein the temperature of the temperature control fluid is controlled as a function of an internal reactor temperature.

2. Apparatus for controlling the temperature of a reactor according to Claim 1, wherein the inner tube (2) of the double tube of the heat exchanger is formed by a single- or double-sided coating with a thermally insulating material, especially a plastic and/or a ceramic, by the construction of the inner tube from a thermally insulating solid material, especially a plastic and/or a ceramic, or by the construction of the inner tube as a double tube, wherein a sealed cavity exists between the two tube walls of the inner double tube.

3. Apparatus for controlling the temperature of a reactor according to Claim 2, wherein the cavity between the two tube walls of the inner double tube of the heat exchanger has been filled with a thermally insulating gas and/or a thermally insulating material or there is a vacuum in the cavity.

4. Apparatus for controlling the temperature of a reactor according to any of Claims 1 to 3, wherein the temperature of the temperature control fluid is controlled as a function of the internal reactor temperature at the point in the reactor where the majority of the chemical reaction proceeds.

5. Apparatus for controlling the temperature of a reactor according to any of Claims 1 to 4, wherein the pressure of the temperature control fluid is controlled as a function of a temperature differential (ΔT) between the internal reactor temperatures at the at least two temperature measurement devices (17, 18) in the reactor.

6. Apparatus for controlling the temperature of a reactor according to any of Claims 1 to 5, wherein the cooling fluid used is water, an aqueous solution, especially an aqueous salt solution, a water-based mixture, especially with one or more alcohols, Marlotherm^{®} or a thermal oil.

7. Apparatus for controlling the temperature of a reactor according to any of Claims 1 to 6, wherein the reactor is a bubble column reactor, a jet loop reactor, a fixed bed reactor or a trickle bed reactor.

8. Apparatus for controlling the temperature of a reactor according to any of Claims 1 to 7, wherein the exothermic reaction is a hydroformylation, a solution polymerization of gaseous monomers, a selective oxidation of organic compounds, for example for production of carboxylic acids, a hydrogenation of C-C or C-X multiple bonds where X = O or N, a Fischer-Tropsch synthesis, if in the slurry phase, a carbonylation reaction, a hydroaminomethylation reaction, a hydrocyanation, a hydrogenation, a hydrosilylation, an oxidation, a pyrolysis, a steam reforming, a dehydrogenation, a dehydration or an oligomerization.

9. Apparatus for controlling the temperature of a reactor according to any of Claims 1 to 8, wherein the heat exchanger system is guided into the reactor from the bottom, from the top or from the side and is correspondingly joined to the base, top or side flange of the reactor, and is preferably guided into the reactor from the top and is joined to the top flange.

10. Use of the apparatus for controlling the temperature of a reactor according to any of Claims 1 to 9 for the cooling of a reactor in which an exothermic reaction can be conducted.

11. Process for conducting exothermic chemical reactions in a reactor, wherein the heat of reaction is removed by the apparatus for controlling the temperature of a reactor according to any of Claims 1 to 9, wherein the cooling fluid that flows through the heat exchanger system does not boil and the temperature differential between the coldest and warmest points in the reactor is less than 13 K.

12. Process according to Claim 11, wherein the exothermic chemical reaction is a hydroformylation with Co, Rh, Ru, Ir, Pd or Fe catalysts.

13. Process according to Claim 12, wherein the temperature differential between the coldest and warmest points in the reactor (on performance of the reaction) is less than 13 K, preferably less than 7.4 K and more preferably less than 5 K.

## Revendications

1. Dispositif de régulation de la température d'un réacteur dans lequel une réaction exothermique ou endothermique peut être effectuée dans un milieu réactionnel, le dispositif comportant un réacteur (6), un système échangeur de chaleur, un récipient de circulation (9) qui contient un liquide de régulation de température avec lequel le système échangeur de chaleur est alimenté, et une pompe (12) qui guide le liquide de régulation de température vers le système échangeur de chaleur par le biais d'une conduite (7) et le ramène au récipient de circulation (9) par le biais d'une conduite (8) et qui règle la quantité de liquide de régulation de température dans le circuit de régulation de température,
le système échangeur de chaleur comprenant au moins un doigt de régulation de température, le doigt de régulation de température étant un double tube qui comprend un tube intérieur (2) par le biais duquel un liquide de régulation de température (1) est introduit dans le double tube et un tube extérieur (3) qui est en contact direct avec le milieu réactionnel et dans lequel le liquide de régulation de température (1) reflue dans l'interstice annulaire ménagé entre le tube intérieur (2) et le tube extérieur (3), le système échangeur de chaleur étant introduit dans le réacteur de sorte que le ou les tubes extérieurs du doigt de régulation de température soient en contact avec le milieu réactionnel,
**caractérisé en ce que** le tube intérieur (2) du double tube est isolé thermiquement, et **en ce que** le dispositif comprend une première vanne d'arrêt (14) permettant de régler la pression du liquide de régulation de température, une deuxième vanne d'arrêt (16) permettant de surveiller et commander la température du liquide de régulation de température et au moins deux dispositifs de mesure de température (17, 18) destinés à mesurer la température interne du réacteur,
la première vanne d'arrêt (14) étant fixée à la conduite (8) menant au récipient de circulation ;
la deuxième vanne d'arrêt (16) étant située sur une conduite de sortie (11) du récipient de circulation par laquelle de la vapeur ou du liquide peut être évacué ; et
la température du liquide de régulation de température étant commandée en fonction d'une température interne du réacteur.

2. Dispositif de régulation de la température d'un réacteur selon la revendication 1, le tube intérieur (2) du double tube de l'échangeur de chaleur étant revêtu sur un ou deux côtés d'une matière thermiquement isolante, notamment une matière synthétique et/ou une céramique, en ce que le tube intérieur est réalisé à partir d'une matière solide thermiquement isolante, en particulier une matière synthétique et/ou une céramique, ou le tube intérieur est réalisé sous la forme d'un double tube, une cavité étanche étant ménagée entre les deux parois du double tube intérieur.

3. Dispositif de régulation de la température d'un réacteur selon la revendication 2, la cavité ménagée entre les deux parois du double tube intérieur de l'échangeur de chaleur étant remplie d'un gaz thermiquement isolant et/ou d'une matière thermiquement isolante ou un vide étant ménagé dans la cavité.

4. Dispositif de régulation de la température d'un réacteur selon l'une des revendications 1 à 3, la température du liquide de régulation de température étant commandée en fonction de la température interne du réacteur au point du réacteur où se déroule la majorité de la réaction chimique.

5. Dispositif de régulation de la température d'un réacteur selon l'une des revendications 1 à 4, la pression du liquide de régulation de température étant commandée en fonction d'une différence de température (ΔT) entre les températures internes du réacteur au niveau des au moins deux dispositifs de mesure de température (17, 18) dans le réacteur.

6. Dispositif de régulation de la température d'un réacteur selon l'une des revendications 1 à 5, le liquide de refroidissement utilisé étant de l'eau, une solution aqueuse, notamment une solution aqueuse de sel, un mélange à base d'eau, notamment comprenant un ou plusieurs alcools, du Marlotherm^{®} ou une huile thermique.

7. Dispositif de régulation de la température d'un réacteur selon l'une des revendications 1 à 6, le réacteur étant un réacteur à colonne à bulles, un réacteur à boucle à jet, un réacteur à lit fixe ou un réacteur à lit ruisselant.

8. Dispositif de régulation de la température d'un réacteur selon l'une des revendications 1 à 7, la réaction exothermique étant une hydroformylation, une polymérisation en solution de monomères gazeux, une oxydation sélective de composés organiques, par exemple pour produire des acides carboxyliques, une hydrogénation de liaisons multiples C-C ou C-X, X = O ou N, une synthèse Fischer-Tropsch si en phase de suspension, une réaction de carbonylation, une réaction d'hydroaminométhylation, une hydrocyanation, une hydrogénation, une hydrosilylation, une oxydation, une pyrolyse, un vaporéformage, une déshydrogénation, une déshydratation ou une oligomérisation.

9. Dispositif de régulation de la température d'un réacteur selon l'une des revendications 1 à 8, le système échangeur de chaleur étant guidé jusque dans le réacteur depuis le bas, depuis le haut ou depuis le côté et étant relié en conséquence à la bride inférieure, supérieure ou latérale du réacteur, de préférence étant guidé depuis le haut jusque dans le réacteur et étant relié à la bride supérieure.

10. Utilisation du dispositif de régulation de la température d'un réacteur selon l'une des revendications 1 à 9 pour refroidir un réacteur dans lequel une réaction exothermique peut être effectuée.

11. Procédé de réalisation de réactions chimiques exothermiques dans un réacteur, la chaleur de réaction étant dissipée par le dispositif de régulation de la température d'un réacteur selon l'une des revendications 1 à 9, le liquide de refroidissement circulant dans le système échangeur de chaleur ne venant pas à ébullition et la différence de température entre le point le plus froid et le point le plus chaud du réacteur étant inférieure à 13 K.

12. Procédé selon la revendication 11, la réaction chimique exothermique étant une hydroformylation avec des catalyseurs Co, Rh, Ru, Ir, Pd ou Fe.

13. Procédé selon la revendication 12, la différence de température entre le point le plus froid et le point le plus chaud du réacteur (lorsque la réaction est en cours) étant inférieure à 13 K, de préférence inférieure à 7,4 K et de manière particulièrement préférée inférieure à 5 K.
